# EUROPÄISCHE PATENTANMELDUNG

(11) **EP 1 415 653 A2**
(43) Veröffentlichungstag der Anmeldung: **06.05.2004**
(21) Anmeldenummer: 03025324.9
(22) Anmeldetag: 03.11.2003
(51) Int. Cl.: A61K 31/57, A61K 31/585, A61K 31/56, A61P 5/08, A61P 25/00, A61P 25/24, A61P 35/00, A61P 35/04, A61P 5/00, A61P 15/08, A61P 25/32, A61P 13/08, A61P 43/00

(54) **Verwendung von Progesteron oder einem Progesteronrezeptor-Agonisten zur Hemmung der Steroidsynthese**

(30) Priorität: 01.11.2002 DE 10251028
(71) Anmelder: Jenapharm GmbH & Co. KG, 07745 Jena (DE)
(72) Erfinder: Paust, Hans-Joachim, 22339 Hamburg (DE); Mukhopadhyay, Amal K., 22523 Hamburg (DE); Patchev, Vladimir, 07749 Jena (DE)
(74) Vertreter: UEXKÜLL & STOLBERG

(57) **Zusammenfassung**

Die vorliegende Erfindung betrifft die Verwendung von Progesteron oder einem Progesteronrezeptor-Agonisten zur Herstellung eines Arzneimittels zur Hemmung der Steroidsynthese, insbesondere zur Hemmung der Expression des "steroidogenic acute regulatory protein" (StAR-Protein).

## Beschreibung

Die vorliegende Erfindung betrifft die Verwendung von Progesteron oder einem Progesteronrezeptor-Agonisten zur Herstellung eines Arzneimittels zur Hemmung der Steroidsynthese, insbesondere zur Hemmung der Expression des "steroidogenic acute regulatory protein" (nachfolgend als StAR bezeichnet).

Progesteron oder 4-Pregnen-3,20-dion ist das wichtigste natürliche Gestagen und wird vorwiegend im Corpus luteum und in der Placenta gebildet. Beim Mann wird dieses Hormon in der Nebenierenrinde und im Hoden gebildet.

Progesteron ist ein Steroidhormon und wirkt zusammen mit den Östrogenen auf die Fortpflanzungsorgane, wobei es an der Regulation nahezu aller weiblicher Reproduktionsfunktionen beteiligt ist. So bestimmt dieses Hormon die Lutealphase des Menstruationszyklus, bereitet den Organismus auf eine Schwangerschaft vor, schafft wichtige Voraussetzungen für Konzeption und Nidation und besitzt unabhängig von Österogenen einen thermogenetischen Effekt.

Die Bildung der Steroidhormone ist für das menschliche Überleben und die Funktion der Fortpflanzung essentiell. Die Biosynthese der Steroidhormone kann unter basalen Bedingungen oder mittels akuter oder chronischer Kontrolle durch trophische Hormone erfolgen, welche in der Hirnanhangsdrüse gebildet werden (Menon und Gunaga, 1974; Waterman and Keney, 1996). Die Bindung trophischer Hormone an den 7-Transmembran-Domänen G-Protein gekoppelten Rezeptor (seven-transmembrane-domain G-protein coupled receptor; 7 TM GPCR) steigert die Aktivität der Adenylatzyklase, was zur Bildung von cAMP führt. cAMP fördert die Steroidbildung durch Induzierung steroidogener Gene (Menon und Gunaga, 1974, Waterman and Keney, 1996).

Steroide werden von spezialisierten steroidogenen Zellen in der Nebenniere, dem Eierstock, der Placenta, dem Hoden und dem Hirn synthetisiert. Obwohl Steroidhormone verschiedene physiologische Wirkungen aufweisen, beginnt die Biosynthese aller Steroide mit der Umwandlung von Cholesterin zu Pregnenolon. Diese Reaktion wird durch das Cytochrom P450 Nebenkettenspaltungsenzym ("cytochrome P450 side-chain-cleavage enzyme" oder P450 sec) katalysiert, das auf der Matrixseite der inneren mitochondrialen Membran lokalisiert ist (Farkash et al., 1986). Viele Jahre war man davon ausgegangen, daß die Aktivität des P450 Enzyms der begrenzende Schritt bei der Steroidherstellung ist. Bei einer unzureichenden Menge des Substrats Cholesterin kann die Aktivität dieses Enzyms tatsächlich begrenzendend wirken. Es wurde jedoch später gefunden, daß die Aktivität des Enzyms nicht der tatsächlich regulierende Schritt ist, sondern vielmehr der Transport des Substrates Cholesterol zu der inneren mitochondrialen Membran und zu dem P450 sec (Brownie et al., 1972, Simpson et al., 1979, Crivello und Jefcoate, 1980, Privalle et al., 1983).

Bei diesen Untersuchungen bestand eine grundlegende Beobachtung darin, daß die Synthese neuer Proteine eine absolute Voraussetzung für die Regulation der Steroidsynthese ist (Ferguson, 1962, 1963). Diese Feststellung beruhte auf der Beobachtung, daß Proteinsyntheseinhibitoren auch die durch trophische Hormone, wie das Adreno-Corticotrope Hormon (ACTH) und das Luteinisierende Hormon (LH), induzierte Steroidbildung hemmten. Weitere Studien konnten zeigen, daß die Hemmung der Proteinsynthese keine Wirkung auf den Transport des zellulären Cholesterins zu der äußeren mitochondrialen Membran hatte. Der Transport des Substrats von der äußeren Membran zu der inneren mitochondrialen Membran war jedoch vollständig gehemmt (Privalle et al., 1983, Ohno et al., 1983).

Folglich wurde ein regulatorisches Protein postuliert, das den Transport des Cholesterins von der äußeren mitochondrialen Membran über den wässrigen, inneren Membranbereich zu der inneren Membran bewirkt. Dieser Transport kann nicht ohne einen Transportvermittler erfolgen, da die hydrophobe Natur des Cholesterins eine Übermittlung zu dem P450 sec nicht in den Mengen möglich wäre, die für die beobachtete Menge der Steroidsynthese nach hormonaler Stimulation notwendig sind. Es gibt mittlerweile überzeugende Anzeichen dafür, daß das StAR-Protein ("steroidogenic acute regulatory protein") dieser Transportvermittler ist und somit die Steroidbildung reguliert.

StAR wurde ursprünglich von Orne-Johnson und Kollegen beschrieben (Brownie et al., 1972). Es wurde als ein schnell induzierbares 30 kDa Phosphoprotein in ACTH-behandelten Ratten und Nebennierenzellen der Maus, sowie in LH-behandelten Zellen des Corpus luteum der Ratte und Leydig Zellen der Maus identifiziert. Später wurde es in mittel Hormon stimulierten MA-10 Maus Leydig Tumorzellen durch Stocco und Kollegen beschrieben (Clarke et al., 1994, Stocco & Clarke, 1996, 1997).

Das Protein wurde in Mitochondrien lokalisiert und besteht aus verschiedenen Formen eines unter Gonadotropin-Einfluss neu synthetisierten 30 kDa Proteins. Zusätzlich zu dem 30 kDa Protein wurden Vorläuferformen dieses Proteins mit 37 kDa gefunden, welche unterschiedliche N-terminale Signalsequenzen aufwiesen (Epstein et al., 1991, Stocco und Sodeman, 1991).

Die für das 37 kDa mitochondriale Protein kodierende cDNA wurde kloniert und mittels transienter Transfektionsversuche in MA-10 und COS-1 Zellen exprimiert, welche nach Transfektion zur Steroidbildung befähigt waren. Diese Vorgehensweise führte zu einer mehrfachen Steigerung der Konversion von Cholesterin zur Pregnenolon (Stocco und Clarke, 1996, Clarke et al., 1994, Sugawara et al., 1995, Lin et al., 1995).

Diese Ergebnisse belegen eine unmittelbare Ursache-Wirkungs-Beziehung zwischen den 37 bzw. 30 kDa Proteinen und der hormonregulierten Steroidsynthese. Ein weiterer Hinweis für eine essentielle Beteiligung des StAR in der Steroidbildung stammt aus der Beobachtung, daß Mutationen in dem StAR Gen der Grund des unter Umständen tödlichen Zustands ist, der als congenitale lipoide Nebennierenhyperplasie (lipoide CAH) bezeichnet wird (Lin et al., 1995).

Patienten mit dieser Erkrankung sind nicht in der Lage, ausreichende Mengen an Steroiden zu erzeugen. Sie weisen eine exzessive Menge an Cholesterin und Cholesterinester in der Nebenniere und in testikulären steroidbildenden Zellen auf. Die Patienten können nur durch eine geeignete Steroidersatztherapie überleben.

Im Stand der Technik sind StAR Knockout Mäuse erzeugt worden, die einen Phenotyp aufweisen, welcher im wesentlichen dem der lipoiden CAH entspricht (Caron et al., 1997). Die biochemischen und genetischen Untersuchungen belegen somit übereinstimmend, daß StAR eine wesentliche Rolle bei der Steroidhormon-Biosynthese in Geweben der Nebenniere und der Gonaden aufweist. Testikuläre Leydig-Zellen sind auf eine chronische Stimulierung mittels LH für den Erhalt ihrer Struktur und der steroidbildenden Funktion angewiesen (Saez, 1994). Während der Alterung wird die Fähigkeit der Leydig-Zellen verringert, Steroide zu bilden (Luo et al., 1996). Der Vorgang des Alterns umfaßt die Bildung reaktiver Sauerstoffradikale, welche Lipide, Proteine und/oder DNA schädigen (Stadtman, 1992). Schädigungen durch reaktive Sauerstoffspezies (ROS) führen gegebenenfalls zu einer funktionalen Seneszenz. Es konnte gezeigt werden, daß diese ROS wesentliche Bestandteile des Steroidbiosyntheseweges schädigen (Quinn und Payne, 1984, 1985). Ferner wurde beobachtet, daß alternde Leydig-Zellen eine gesteigerte Herstellung von Progesteron und eine verringerte Bildung von Testosteron aufweisen.

Es gibt eine Vielzahl von Erkrankungen, bei denen die adrenale Steroidsynthese durch pathologisch aktivierte Stimulationsmechanismen abnorm gesteigert ist. Eine natürliche und sichere Kontrolle der Steroidbiosynthese wäre für die Therapie dieser Krankheitszustände und auch für andere medizinische Zwecke wünschenswert.

Demgemäß liegt der vorliegenden Erfindung die Aufgabe zugrunde, Wirkstoffe bereitzustellen, welche die Steroidbiosynthese auf möglichst natürliche Art und Weise hemmen und somit in besonderem Maße für die Behandlung pathologischer Zustände geeignet sind.

Dieses Problem wurde erfindungsgemäß durch die Verwendung von Progesteron oder einem Progesteronrezeptor-Agonisten zur Herstellung eines Arzneimittels zur Hemmung der StAR Genexpression gelöst.

Erfindungsgemäß wurde somit überraschenderweise festgestellt, daß ein Steroidhormon, nämlich Progesteron oder ein Progesteronrezeptor-Agonist, in besonderem Maße für die Hemmung der Expression des StAR Gens geeignet ist. Progesteron und Progesteronrezeptor-Agonisten bieten somit natürliche, sichere und hochwirksame Hemmstoffe für die StAR Expression, was als Folge zu einer Hemmung der Steriodsynthese führt, die auch unabhängig von der Inhibierung supra-adrenaler Faktoren ist (Cortico- oder Gonadotropine).

In Übereinstimmung mit der üblichen Bezeichung wird der Begriff Progesteron (INN) in der vorliegenden Anmeldung dazu verwendet, um auf die Verbindung 4-Pregnen-3,20-dion (IUPAC) zu verweisen.

Ferner bezeichnet der Begriff "Progesteronrezeptor-Agonist" im Rahmen der vorliegenden Erfindung eine Verbindung, die eine meßbare spezifische Bindung mit dem Progesteron-Rezeptor eingeht und nach erfolgter Bindung an den Rezeptor eine signifikante Veränderung der Transkription definierter gestagen-responsiver Gene herbeiführt. Beispiele für ein Progesteronrezeptor-Agonisten sind Gestagene, wie beispielsweise R5020 und Drospirenon.

Eine Hemmung der StAR Genexpression liegt vor, wenn in einer Probe eines behandelten Subjektes eine Verringerung der Expression des StAR Gens um mindestens 20% im Vergleich zu einer entsprechenden Probe desselben Subjektes vor der Behandlung oder im Vergleich zu einer entsprechenden Probe eines unbehandelten Subjektes feststellbar ist. Bei der Probe kann es sich um eine Gewebeprobe handeln.

Die Verringerung der Expression des StAR Gens erfolgt um mindestens 20%, vorzugsweise um mindestens 30%, besonders bevorzugt um mindestens 50%. Vom Umfang der Erfindung umfaßt ist selbstverständlich auch die vollständige Hemmung der Expression des StAR Gens, also eine Hemmung, welche die Menge der StAR Transkripte bis unter die Nachweisgrenze verringert.

Die Hemmung der Genexpression kann durch quantitative Bestimmung der StAR mRNA oder durch Ermittlung der StAR Proteinmenge in Geweben oder Proben festgestellt werden. Eine Vielzahl von Verfahren zur quantitativen Bestimmung von mRNA, beispielsweise Northern Blot, RT-PCR, Nukleinsäure-Chips, etc., und zur quantitativen Bestimmung von Proteinen, beispielsweise Western Blot, Proteinchips etc., sind dem Durchschnittsfachmann bekannt und können im Rahmen der vorliegenden Erfindung verwendet werden, um eine Hemmung der StAR Genexpression zu ermitteln.

Die vorliegende Erfindung umfaßt die Verwendung von Progesteron oder einem Progesteronrezeptor-Agonisten zur Herstellung eines Arzneimittels zur Hemmung der StAR Genexpression zur Behandlung von Säugetieren, insbesondere zur therapeutischen oder prophylaktischen Behandlung von Menschen.

Gemäß einer besonders bevorzugten Ausführungsform der Erfindung wird Hemmung der StAR Genexpression durch Progesteron oder ein Progesteronrezeptor-Agonist für die Behandlung eines pathologischen Zustands der Steroidsynthese verwendet. Im Rahmen der vorliegenden Erfindung wird ein Zustand als pathologischer Zustand der Steroidsynthese bezeichnet, wenn die basale Steroidsynthese über einen längeren Zeitraum, vorzugsweise über einen Zeitraum von 24 Stunden, um mindestens 25%, vorzugsweise mindestens 30% oder 50%, bezogen auf die Menge der Steroidsynthese eines gesunden Individuums gleichen Alters gesteigert ist. Die in einem gesunden Individuum üblicherweise zu erwartenden Mengen an Steroiden sind dem Fachmann für die einzelnen Steroide unter Berücksichtigung bestimmter Parametern, wie beispielsweise Alter und Geschlecht des Individuums, hinreichend bekannt. Normwerte bzw. Normbereiche dieser Steroidmengen sind insbesondere in dem Lehrbuch von Allolio und Schulte, "Praktische Endokrinologie", XV. Anhang, veröffentlicht.

So kann die erfindungsgemäße Verwendung von Progesteron oder einem Progesteronrezeptor-Agonisten insbesondere zur Behandlung eines pathologischen Zustands, bei dem die adrenale Steroidsynthese durch pathologisch aktivierte Stimulationsmechanismen abnorm gesteigert ist, oder eines Zustands verwendet werden, der eine pathologisch erhöhte Aktivität der Nebennierenrinde umfasst, welche aus einer angeborenen oder medikamentös verursachten Glucocorticoid-Rezeptorresistenz resultiert (vgl. beispielsweise Kamilaris und Chrousos "Adrenal diseases", In: Diagnostic Endocrinology, B.C. Decker Inc., 1990, Seite 79-109) . Der pathologische Zustand kann somit beispielsweise chronischer Stress, Alkoholentzug, endogene Depression, ACTHund/oder Gonadotropin-sezernierende Tumoren des Hypophysenvorderlappens bzw. deren Metastasen, ektopisches ACTH-Syndrom (bronchiale Carzinoide), Prostatahyperplasie, Krebs, mikronoduläre adrenale Erkrankung, kongenitale adrenale Hyperplasie (CAH), Pubertas praecox, virilisierendes Syndrom bei Frauen oder polycystisches Ovar mit nachgewiesener Androgen-Hypersekretion sein.

Im Rahmen der vorliegenden Erfindung kann das Progesteron oder der Progesteronrezeptor-Agonist nach beliebigen im Stand der Technik bekannten Verfahren an ein Subjekt verabreicht werden. Die zu verabreichende Menge hängt im Einzelfall von dem Behandlungsziel und dem Wirkstoff ab und kann vom Fachmann mittels Standardverfahren bestimmt werden. So kann die Menge des zu verabreichenden Progesteron oder des Progesteronrezeptor-Agonisten beispielsweise bei einzelnen Indikationen im Bereich von 2-5 mg/Tag liegen.

Es können eine Vielzahl von Verabreichungsformen zur Anwendung kommen, unter denen die orale Verabreichung, beispielsweise in Form einer Tablette, sowie parenterale Techniken wie subkutane, intravenöse und intraperitoneale Injektionen, Katheterisierungen und dergleichen bevorzugt sind. Ferner kann die Verabreichung mittels Hautpflaster oder eine nasale sowie andere Formen der Verabreichung durch die Schleimhaut vorgenommen werden.

Die vorliegende Erfindung betrifft Verfahren zur Herstellung eines Arzneimittels, das Progesteron oder einen Progesteronrezeptor-Agonisten enthält und das zur Hemmung der StAR Genexpression verwendet werden soll. Alle zur Herstellung des Arzneimittels notwendigen Substanzen sind kommerziell erhältlich (z.B. Drospirenon von der Schering AG, Deutschland; R5020 erhältlich als Promegestone von NEN Liefe Science, Boston MA) .

Die Dosierung und Verabreichungsart des Progesteron oder Progesteronrezeptor-Agonisten kann vom Durchschnittfachmann im Hinblick auf die geplante medizinische Indikation durch im Stand der Technik bekannte Verfahren ermittelt werden.

Gemäß einer weiteren Ausführungsform der vorliegenden Erfindung wird die Verwendung von Progesteron oder einem Progesteronrezeptor-Agonisten zur Herstellung eines Arzneimittels zur therapeutischen oder prophylaktischen Behandlung einer Erkankung bereitgestellt, wobei die Erkrankung durch eine erhöhte Steroidmenge in dem Patienten gekennzeichnet ist.

### Material und Methoden

Chemikalien und Reagenzien wurden von folgenden Herstellern bezogen:

Humanes chorionisches Gonadotrophin (hCG) wurde von Boehringer Mannheim (Mannheim, Deutschland) bezogen. Steroide wie Dihydrotestosteron (DHT), Estradiol und Progesteron wurden von Signma (Taufkirchen, Deutschland) erhalten. R5020 NEN Life Sciences und Drospirenon wurden von Jenapharm (Jena, Deutschland) bezogen. Albuminfraktion V (aus bovinem Serum) wurde von Merck (Darmstadt, Deutschland) erhalten. Alle übrigen Reagenzien wurden von kommerziellen Anbietern bezogen und entsprachen dem höchsten Reinheitsgrad.

### Isolation und Aufreinigung von Leydig-Zellen:

Die Hoden von jungen (3 Monate alten) und alten (23-24 Monate alten) Wistar-Ratten (Charles River, Sulzfeld, Deutschland) wurden unmittelbar nach Tötung der Ratten durch Carbondioxid-Anästhesie und zervikaler Dislokation entfernt.

Um Leydig-Zellen aus den Hoden von jungen und alten Ratten zu isolieren und zu reinigen, wurde die dekapsulierte testikulare Masse von gesunden Wistar-Ratten mit Collagenase (0,25 mg/ml) für 30 Min. bei Raumtemperatur, wie bei Mukhopadhyay et al. (1986a,b) beschrieben, behandelt. Die so erhaltene homogene Zellsuspension wurde durch einen Nylonfilter filtriert, um eine ungereinigte Leydig-Zellpräparation zu erhalten. Nach weiterer Aufreinigung mittels eines Percoll-Gradienten wurde die finale Zellzahl der hochgradig gereinigten Leydig-Zellen unter Verwendung einer Neubauer-Kammer bestimmt.

Für die Experimente, in denen cAMP gemessen wurde, verwendete man Aliquots von 100,000 Zellen pro Inkubatinsröhrchen. Die Zellen wurden für 3 Stunden vorinkubiert und anschließend für 30 Min. mit oder ohne Zusatz von verschiedenen Konzentrationen von hCG als Surrogat für LH inkubiert. Um die Auswirkungen der verschiedenen Steroide zu untersuchen, wurden Leydig-Zellen wie beschrieben für 3 Std. Steroiden ausgesetzt. Anschließend wurden die Zellen für 30 Min. mit hCG (3 ng/ml) stimuliert. Die Inkubation wurde durch die Zugabe von absolutem Ethanol (finale Alkoholkonzentration ca. 80%) und nachfolgendem Mischen durch Vortexen abgestoppt. Der Ethanol-Extrakt wurde bis zur Trockenheit eingedampft und das verbliebende wurde in 1 ml MEM, das 0,1% Natriumazid enthielt, gelöst. Die Menge des gebildeten cAMP wurde durch einen spezifischen ELISA bestimmt, der wie in Budnik und Mukhopadhyay (1997) beschrieben durchgeführt wurde. Es können jedoch auch kommerziell erhältliche ELISAs, wie beispielsweise CM 59221 (IBL, Hamburg, Deutschland) oder DE 0355 bzw. DE 0450 (R&D Systems GmbH, Wiesbaden, Deutschland) verwendet werden.

Für Experimente, in denen Testerosteron gemessen wurde, wurden ähnliche Kulturbedingungen wie oben beschrieben verwendet. Es wurden jedoch Aliquots von 50,000 Zellen pro Inkubationsröhrchen verwendet. Die Zellen wurden für 3 Stunden mit verschiedenen Steroiden oder ohne Steroide vorinkubiert, und anschließend für 30 min in Abwesenheit oder in Gegenwart von hCG inkubiert. Die Inkubation wurde durch Zusatz von absolutem Ethanol (endültige Alkoholkonzentration ca. 80%) und nachfolgendem Mischen durch Vortexen abgestoppt. Der ethanolische Extrakt wurde bis zur Trockenheit eingedampft, das verbliebene wurde in 1 ml MEM, das 0,1% Natriumazid enthielt, gelöst. Der Gehalt von Testosteron wurde durch spezifische, kommerziell erhältliche Kits (IBL, Hamburg) bestimmt. Anhand der gemessenen Werte wurde die Testosteron-Menge für 100,000 Zellen errechnet.

### Extraktion von RNA und Northern Hybridisierung

RNA wurde aus 1,5 × 10⁶ hochgradig gereinigten Leydig-Zellen isoliert, die in 12-Wellplatten (750.000 Zellen/well) kultiviert wurden. Die RNA wurde nach einem modifizierten 1-Schritt-Verfahren von Chomczynski und Sacchi (1987) unter Verwendung des small scale Phenol-free RNA isolation kit (Ambion) extrahiert. Die isolierte RNA wurde der Größe nach gelelektrophoretisch in einem 1,2%igen Agarosegel unter Verwendung des Formaldehyd/Morpholinopropansulfonsäure-Verfahrens (Sambrooket al., 1989) aufgetrennt. Der Transfer (Blotten) der RNAaus dem Gel auf eine Nylonmembran (Nytran, Schleicher und Schüll, Dassel, Deutschland) wurde mittels eines nach unten gerichteten Transfers für 4 Stunden durch Kapillartransfer gemäß den Angaben des Herstellers (Ambion, AMS Biotechnology, Wiesbaden, Deutschland) mit Modifikationen von Chomzynski (1992) durchgeführt.

Die für das StAR Protein spezifische Hybridisierungssonde wurde mittels PCR unter Verwendung eines 5'Primers und eines 3'Primers amplifiziert, die in der publizierten cDNA Sequenz (Clark et al., 1994) in den Positionen 339-360 nt und in den Positionen 581-600 nt lokalisiert sind, und in den Klonierungsvektor pCR™II (Invitrogen, Groningen, Niederlande) subkloniert. Um in Leydig-Zellen Transkripte nachzuweisen, die für das StAR Protein kodierten, wurde Antisense RNA in vitro von einem cDNA Fragment (1 µg) transkribiert, das von der isolierten Plasmid DNA amplifziert wurde, welche ein 262-Basenpaar Insert des StAR Proteingens unter der Transkriptionskontrolle des T7 Promoters enthielt. Dabei wurde ein M13 Forward Primer und der spezifische 5' Primer des StAR Proteins verwendet. Die Transkriptionsreaktion wurde unter Bedingungen, die dem Ambion Protokoll entsprachen, unter Verwendung von [α-³²P]UTP (ca. 800 Ci/mmol) (erhältlich von Amersham-Pharmacia Biotech, Freiburg, Deutschland) durchgeführt. Für die Hybridisierung wurden 1×10⁶ cpm/ml für 18 Stunden bei 68°C verwendet, gefolgt von Waschschritten mit niedriger und hoher Stringenz bei 68°C in 2×SSC, 0,1% SDS und 0,1×SSC, 0,1%SDS. Die Blots wurden einem autoradiographischem Film oder der Phospho-Imaging-Detektion mit Hilfe der Storm 860-Apperatur (Molecular Dynamics, Amersham Biosciences, Freiburg, Deutschland) ausgesetzt.

### Beispiele

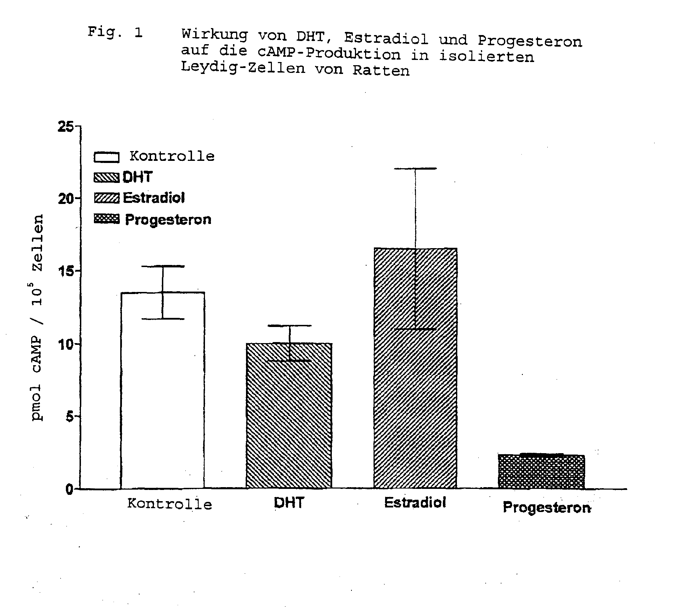

Leydig-Zellen wurden 3 verschiedenen Steroiden für 3 Stunden ausgesetzt (Fig.1). Anschließend wurden die Zellen über 30 Minuten mit hCG (humanes Choriongonadotropin; 3 ng/ml) als Surrogat für LH stimuliert.

Die Menge des gebildeten cAMP wurde durch die Verwendung eines spezifischen ELISA ermittelt.

Aus den gewonnenen Daten wird klar, daß die Gegenwart von Progesteron die Leydig-Zellen zu einer deutlichen Hemmung der cAMP Reaktion auf gonadotropine Stimulierung bewirkt. Keines der anderen in diesem Experiment eingesetzten Steroide wies eine ähnliche Wirkung auf die hCG-stimulierte cAMP Akkumulation auf.

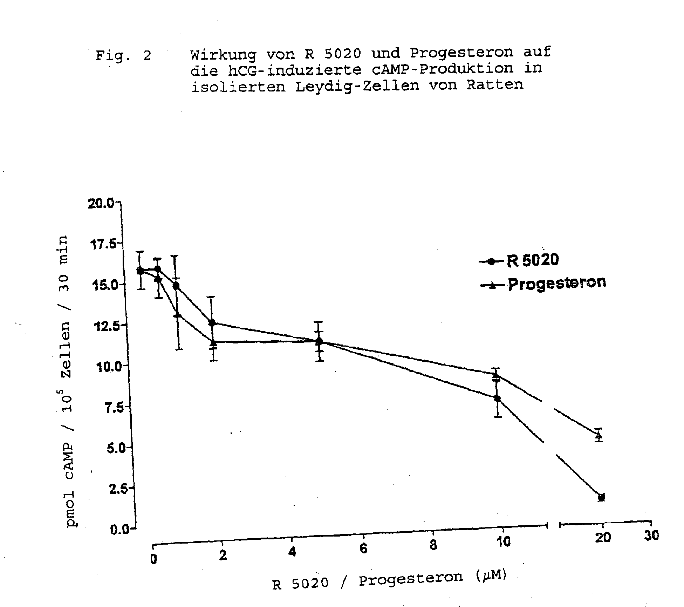

Eine Dosis-Wirkungskurve für die Wirkung des Progesterons auf die Leydig-Zellen im Hinblick auf die cAMP Bildung wurde erstellt (Fig. 2). Die in Fig. 2 dargestellten Daten zeigen klar, daß ein dosisabhängiger Hemmeffekt des Progesterons auf hCG-induzierte cAMP Bildung in Leydig-Zellen beobachtet werden kann.

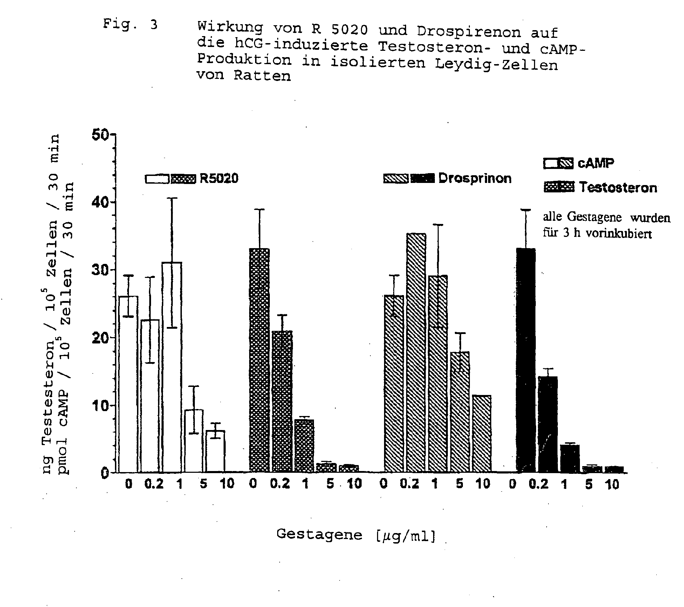

Obwohl Progesteron bei einer Konzentration von 0,1 µmol/l (10⁻⁷ mol/l) fast keine Wirkungen aufwies, zeigten höhere Konzentrationen, bis zu 2,5 µmol/l Progesteron, klar hemmende Wirkungen. Diese Wirkung des Progesterons wird auch durch andere Gestagene, wie R5020 (Fig. 2 und 3) und Drospirenon (Fig. 3), ausgelöst. Die Dosis-Wirkungskurve entsprach der des Progesterons (Fig. 2). R5020 und Drospirenon reduzierten die in den Zellen als Reaktion auf gonadotrope Stimulation gebildete cAMP Menge drastisch.

Sowohl R5020 und Drospirenon sind struktuell deutlich von Testosteron verschieden. Es sollte festgestellt werden, ob diese beiden Verbindungen ebenfalls die hCG-induzierte Testosteronsynthese in Leydig-Zellen hemmen. In Fig. 3 werden Daten eines Versuchs gezeigt, der unter ähnlichen Bedingungen wie in Fig. 2 durchgeführt wurde. In diesem Fall wurden die Zellen mit verschiedenen Konzentrationen von R5020 und Drospirenon über 3 Stunden behandelt, gefolgt von einer Stimulierung mit hCG (5 ng/ml) für 3 Stunden und die Menge des Testosterons, das im Medium akkumulierte, wurde gemessen. Die Ergebnisse zeigen eindeutig, daß beide Verbindungen in der Lage waren, auch die Testosteronherstellung zu hemmen.

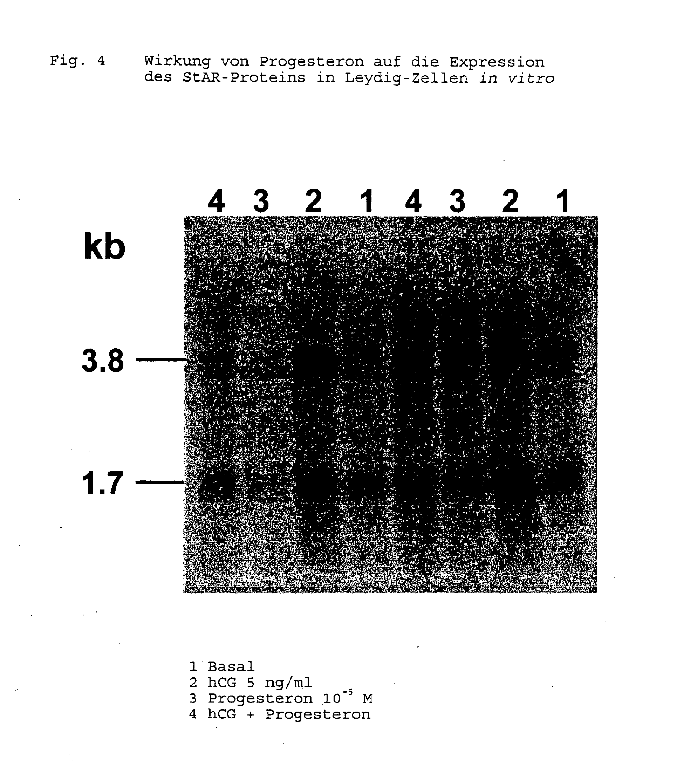

Da die oben genannten Gestagene offensichtlich sowohl hemmende Wirkungen auf cAMP als auch auf die Steroidherstellung in Gonadotropin-stimulierten Leydig-Zellen aufweisen, wurden die hemmenden Wirkungen von Progesteron auf die Menge der StAR Proteinexpression analysiert. Um die Wirkung des Progesterons auf die Expression des StAR Proteins zu untersuchen, wurden Leydig-Zellen für 3 Stunden mit oder ohne Progesteron vorinkubiert. Anschließend wurden die Zellen weiter mit hCG über 3 Stunden inkubiert (5ng/ml). Nach Beendigung der Inkubation wurde die Gesamt RNA extrahiert und die Expression des StAR Proteins wurde unter Verwendung von Northern Blots analysiert. Die Ergebnisse zweier repräsentativer Versuche, von denen jedes mit unabhängigen Zellproben durchgeführt wurde, werden in Fig. 4 gezeigt. Bahn 1 zeigt Zellen, die nicht behandelt wurden, Bahn 2 zeigt Zellen, die mit hCG alleine behandelt wurden. Es ist deutlich, daß zwei Transkripte des StAR Proteins, eins mit 3,8 kb und ein weiteres mit 1,7 kb Länge, gefunden wurden. Die Expression der Transkripte wird erwartungsgemäß durch die Stimulierung mit hCG verstärkt.

Die Vorbehandlung mit Progesteron konnte die Expression des StAR Proteins bei solchen Zellen, die nicht mit einem Gonadotropin behandelt wurden (Bahn 3), nicht wesentlich beeinflussen. Diese Zellen wurden im Rahmen des vorliegenden Experimentes als Negativ-Kontrolle verwendet; es ließ sich somit zeigen, daß Progesteron die konstitutive Expression des StAR-Proteins nicht beeinflußt. Nur unter Bedingungen einer Über-Stimulation der Steroidsynthese kann eine hemmende Wirkung nachgewiesen werden, d.h. erst wenn die Zellen mit supra-adrenalen Faktoren (Cortico- oder Gonadotropine) stimuliert werden, wird die StAR-Expression gehemmt.

Eine Verringerung des Signals in Bahn 3 im Verhältnis zu Bahn 1 war jedoch erkennbar. Wie jedoch in Bahn 4 gezeigt, führte die Vorbehandlung mit Progesteron zu einer deutlichen Reduzierung der hCG stimulierten Mengen des StAR Proteins (vgl. Bahn 4 und Bahn 2).

Die hier beschriebenen Versuche belegen daher eindeutig, daß die Behandlung mit Progesteron eine Hemmung der hCG-stimulierten StAR Proteinexpression in primären Leydig-Zellen bewirkt. Erstmals konnte somit auf molekularer Ebene gezeigt werden, daß Progesteron die Expression der für das StAR Protein kodierenden Transkripte in Leydig-Zellen hemmt.

### Literaturhinweise

Allolio und Schulte, "Praktische Endokrinologie", Urban & Fischer Verlag, 1996, XV. Anhang, 730-736;
Brownie et al., Biochem Biophys Res Commun 46; 1972, 483-490; Budnik und Mukhopadhyay, FEBS Letters 419, 1997, 4-8;
Caron et al., Proc Natl Acad Sci USA 94; 1997, 11540-11545;
Chomczynski und Sacchi, Anal. Biochem. 162, 1987, 156-159;
Chomczynski, P., Anal. Biochem. 201; 1992, 134-139;
Clark et al., J Biol Chem 269; 1994, 8314-28322;
Crivello et al., J Biol Chem 255; 1980, 8144-8151;
Epstein und Orme-Johnson 1991, J. Biol. Chem., 266:19739-19745.
Farkash et al., 1986, Endicrinology 118;1353-1365;
Ferguson JJ, 1962, Biochim Biophys Acta 57:616-617;
Ferguson JJ 1963, J Biol Chem 238:2754-2759;
Kamilaris und Chrousos "Adrenal diseases", In: Diagnostic Endocrinology, B.C. Decker Inc., 1990, Seite 79-109)
Lin et al., 1995 Science 267:1828-1831;
Luo et al., 1996, J. Andrology 17:509-515;
Menon et al., 1974, Fertil Steril 25:732-750;
Mukhopadhyay et al., Biochem J., 1986a, 239:463-467;
Mukhopadhyay et al., FEBS Lett., 1986b, 202:111-116;
Ohno et al., 1983, Endocrinology 30:355-228;
Privalle et al., 1983, Proc Natl Aad Sci USA 80:702-706;
Quinn et al., 1984, J Biol Chem 259:4130-4135;
Quinn et al., 1985, J Bio Chem 260:2092-2099;
Saez JM 1994, 1994, Endocr Rev 15:574-626;
Sambrook et al., vol.1, 7·37, 7·39-7·52. Nolan, C.(Ed), Cold Spring Harbor Laboratory Press, Cold Spring Harbor, New York; Simpson et al., J 1979, Biol Chem 253-3135-3139;
Stadtman, 1992, Science 257:1220-1224;
Stocco et al., 1991, J Biol Chem 266:19731-19738;
Stocco et al., 1996, Endocr Rev 17:221-244;
Stocco et al., 1997, Steroids 62:29-36;
Sugawara et al., 1995, Proc Natl Acad Sci USA 92:4778-4782;
Waterman et al., 1996, Vit horm 52:129-1548.

## Patentansprüche

1. Verwendung von Progesteron oder einem Progesteronrezeptor-Agonisten zur Herstellung eines Arzneimittels zur Hemmung der StAR-Genexpression.

2. Verwendung gemäß Anspruch 1, bei der die Hemmung der StAR Genexpression zur Behandlung von Säugetieren, insbesondere zur therapeutischen oder prophylaktischen Behandlung eingesetzt wird.

3. Verwendung gemäß Ansprch 1, bei der die Hemmung der StAR Genexpression durch Progesteron oder ein Progesteronrezeptor-Agonist für die Behandlung eines pathologischen Zustands der Steroidsynthese eingesetzt wird.

4. Verwendung gemäß einem der vorstehenden Ansprüche, bei der der Progesteronrezeptor-Agonist R5020 oder Drospirenon ist.

5. Verwendung gemäß einem der vorstehenden Ansprüche, bei der die Hemmung der StAR Genexpression eine Verringerung der Expression des StAR Gens um mindestens 20%, vorzugsweise um mindestens 30%, besonders bevorzugt um mindestens 50%, umfaßt.

6. Verwendung gemäß einem der vorstehenden Ansprüche, zur Behandlung eines Zustands, bei dem die adrenale Steroidsynthese durch pathologisch aktivierte Stimulationsmechanismen abnorm gesteigert ist, oder zur Behandlung eines Zustands, der eine pathologisch erhöhte Aktivität der Nebennierenrinde umfasst, welche aus einer angeborenen oder medikamentös verursachten Glucocorticoid-Rezeptorresistenz resultiert.

7. Verwendung gemäß Anspruch 6, bei der der pathologische Zustand ausgewählt ist aus der Gruppe, bestehend aus: chronischer Stress, Alkoholentzug, endogene Depression, ACTH- und/oder Gonadotropin-sezernierende Tumoren des Hypophysenvorderlappens bzw. deren Metastasen, ektopisches ACTH-Syndrom (bronchiale Carzinoide), Prostatahyperplasie, Krebs, mikronoduläre adrenale Erkrankung, kongenitale adrenale Hyperplasie (CAH), Pubertas praecox, virilisierendes Syndrom bei Frauen und polycystisches Ovar mit nachgewiesener Androgen-Hypersekretion.

8. Verwendung gemäß einem der vorstehenden Ansprüche, bei der die Behandlung bei Säugetieren, insbesondere beim Menschen eingesetzt wird.

9. Verwendung gemäß einem der vorstehenden Ansprüche, bei der das Progesteron oder der Progesteronrezeptor-Agonist oral, beispielsweise in Form einer Tablette, oder parenteral, beispielsweise durch subkutane, intravenöse oder intraperitoneale Injektion oder Katheterisierung verabreicht wird.
